# EUROPEAN PATENT SPECIFICATION

(11) **EP 2 016 889 B1**
(45) Date of publication and mention of the grant of the patent: **13.10.2010**
(21) Application number: 08012953.9
(22) Date of filing: 17.07.2008
(51) Int. Cl.: A61B 3/12

(54) **Fundus imaging apparatus**
Vorrichtung zur Abbildung des Augenhintergrunds
Appareil d'imagerie du fond oculaire

(30) Priority: 19.07.2007 JP 2007188290
(43) Date of publication of application: 21.01.2009
(73) Proprietor: Nidek Co., Ltd., Gamagori-shi Aichi 443-0038 (JP)
(72) Inventor: Akita, Junichi, Gamagori-shi Aichi-ken 443-0038 (JP)
(74) Representative: Prüfer & Partner GbR European Patent Attorneys

(56) References cited:
- EP-A- 1 188 411
- EP-A- 1 698 273
- WO-A-2006/016366
- DE-A1- 4 126 329

## Description

### BACKGROUND OF THE INVENTION

### 1. Field of the Invention

The present invention relates to a fundus imaging apparatus arranged to image an eye fundus for observation and/or examination thereof.

### 2. Description of Related Art

As a fundus imaging apparatus for imaging an eye fundus by scanning light on the fundus, an apparatus arranged to obtain at least two kinds of fluorescent fundus images at almost the same time has been proposed. In this apparatus, a first filter allowing passage of first fluorescence generated in the fundus by scan of first excitation light and a second filter allowing passage of second fluorescence generated in the fundus by scan of second excitation light are continuously selectively disposed in an optical path of an imaging optical system (see UP 7198367 (JP2006-239196A)).

One of such apparatus is provided with a filter disc formed with an opening between the first and second filters. The opening will be fixedly disposed in the optical path of the imaging optical system for alignment (positioning) between an examinee's eye and the apparatus, normal fundus imaging, and other cases.

Meanwhile, in the case of fluorescence fundus imaging, the fluorescence generated in the fundus is very weak. A photo-receiving element is therefore preferably set to provide high sensitivity in order to obtain a clear fluorescent fundus image. However, if it is arranged to continuously obtain at least two kinds of fluorescent fundus images by rotation of the above filter disc having the opening, the opening will be disposed in the optical path during changeover from one filter to the other. Thus, excitation light having been reflected by the fundus and passing through the opening falls on the photo-receiving element set with the high sensitivity and hence the photo-receiving element is likely to become saturated. Consequently, fluorescent fundus images may not be obtained for a while.

### SUMMARY OF THE INVENTION

The present invention has an object to provide a fundus imaging apparatus capable of obtaining at least two kinds of clear fluorescent fundus images at almost the same time.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the above object, the present invention provides a fundus imaging apparatus comprising: a beam emitter adapted to simultaneously emit a first laser beam and a second laser beam having a different wavelength from the first beam; an irradiation optical system having a beam scanner for scanning the emitted first and second beams in two dimensions on a fundus, the irradiation optical system being adapted to irradiate the emitted first and second beams onto the fundus; a filter disc having a first filter which intercepts the first and second beams reflected by the fundus and second fluorescence from the fundus by irradiation of the second beam and transmits first fluorescence from the fundus by irradiation of the first beam, a second filter which intercepts the first and second beams reflected by the fundus and the first fluorescence from the fundus and transmits the second fluorescence from the fundus, and an opening; an imaging optical system having the filter disc and a photo-receiving element; a rotator for rotating the filter disc to dispose one of the first filter, the second filter, and the opening in an optical path of the imaging optical system; a sensor for detecting a rotation angle of the filter disc; control means for controlling the beam emitter to simultaneously emit the first and second beams at respective predetermined powers; and image processing means for obtaining a first fluorescent fundus image based on a received light signal of the first fluorescence from the photo-receiving element and obtaining a second fluorescent fundus image based on a received light signal of the second fluorescence from the photo-receiving element, characterized by the control means being adapted to control at least one of the beam emitter, the irradiation optical system, and the imaging optical system based on a detection signal of the sensor to reduce respective light amounts of the first and second beams to be received by the photo-receiving element while the opening is disposed in the optical path of the imaging optical system.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.
Fig. 1 is a schematic configuration view of an optical system of a fundus imaging apparatus in a preferred embodiment of the present invention;
Fig. 2 is a schematic configuration view of a beam emitting part;
Fig. 3 is a schematic configuration view of a filter disc;
Fig. 4A is a graph showing a spectral transmission property of a first filter used for infrared-fluorescence fundus imaging;
Fig. 4B is a graph showing a spectral transmission property of a second filter used for visible-fluorescence fundus imaging;
Fig. 5 is a schematic block diagram of a control system of the fundus imaging apparatus; and
Fig. 6 is a graph to explain control of each power of an infrared, first laser beam and a visible, second laser beam according to a rotation angle of the filter disc.

### DETAILED DESCRIPTION OF THE PREFERRED EMBODIMENTS

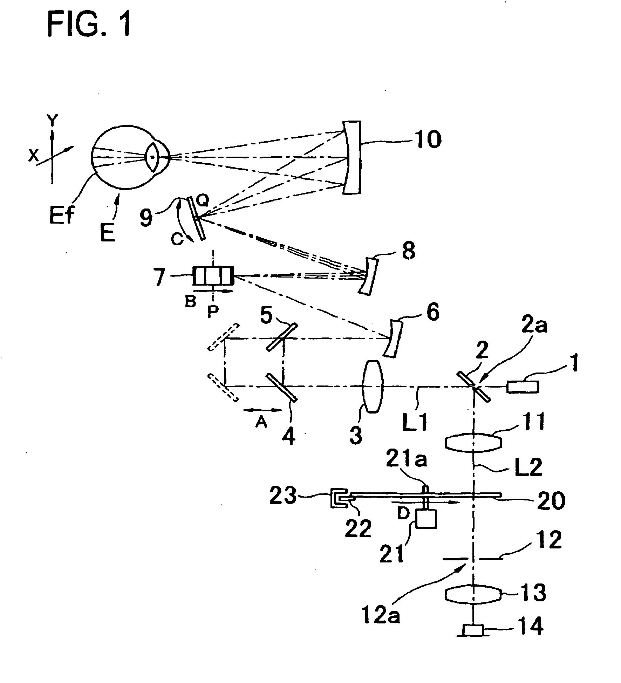

A detailed description of a preferred embodiment of the present invention will now be given referring to the accompanying drawings. Fig. 1 is a schematic configuration view of an optical system of a fundus imaging apparatus in this embodiment.

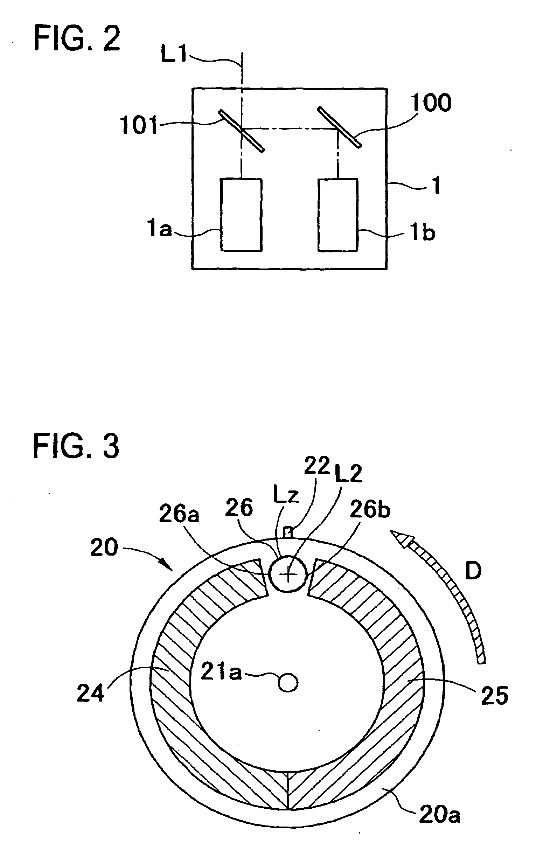

A beam emitting part (a beam emitter) 1 includes a first laser source 1a which emits a first laser beam of wavelengths in an infrared region, a second laser source 1b which emits a second laser beam of wavelengths in a visible region, a total reflection plane mirror 100, and a dichroic mirror 101 which transmits infrared light and reflects visible light, as shown in Fig. 2. In the present embodiment, the first laser source 1a emits the first beam having a wavelength of about 790 nm. The second laser source 1b emits the second beam having a wavelength of about 490 nm. The first beam emitted from the first laser source 1a passes through the dichroic mirror 101 to travel along an optical axis L1. The second beam emitted from the second laser source 1b is reflected by the mirror 100 and the dichroic mirror 101 and thereby made coaxial with the first beam to travel along the optical axis L1.

The first beam and the second beam emitted from the beam emitting part 1 pass through an opening 2a of a perforated mirror 2 (through a substantial center of which the optical axis L1 passes) and a lens 3, and are reflected by total reflection plane mirrors 4 and 5 and a total reflection concave mirror 6 in turn, and then fall on a polygon mirror 7. The first and second beams reflected by the polygon mirror 7 are reflected by a total reflection concave mirror 8 and fall on a galvano mirror 9. The first and second beams reflected by the galvano mirror 9 are reflected by a total reflection concave mirror 10 and focused on a fundus Ef of a patient's eye E. The above optical members constitute an irradiation optical system for the first and second beams.

The mirrors 4 and 5 are arranged to be movable in a direction indicated by an arrow A in order to change the length of optical path for focus adjustment (diopter movement). The polygon mirror 7 is rotated about an axis P in a direction indicated by an arrow B in order to scan the first and second beams on the fundus Ef in a direction indicated by an arrow X. The galvano mirror 9 is swung (rotated) about an axis Q in a direction indicated by an arrow C in order to scan the first and second beams on the fundus Ef in a direction indicated by an arrow Y perpendicular to the X direction. With the above beam scanners, the first and second beams are scanned on the fundus Ef in two dimensions.

The first and second beams scanned on the fundus Ef and reflected therefrom reversely travel along the above mentioned irradiation optical system and are reflected by a surface surrounding the opening 2a of the perforated mirror 2 to travel along an optical axis L2. The opening 2a of the perforated mirror 2 is substantially conjugated with a pupil of the eye E through the lens 3. The first and second beams reflected by the perforated mirror 2 pass through a lens 11 and a filter disc 20 to come into focus on a pinhole 12a of a pinhole plate 12 (through a substantial center of which the optical axis L2 passes). The pinhole 12a is substantially conjugated with the fundus Ef through the lens 11. The first and second beams focused on the pinhole 12a pass through a lens 13 to fall on a photo-receiving element 14 having sensitivity to light of wavelengths in a visible region and an infrared region. The above optical members constitute an imaging optical system.

The diameter of the pinhole 12a in the present embodiment is fixed. Alternatively, the pinhole 12a may be formed with a variable diameter to allow changes in contrast and luminance of an image of the fundus Ef to be obtained. The photo-receiving element 14 in the present embodiment is APD (avalanche photodiode), but it may be a well-known photo-receiving element or the like.

The filter disc 20 is disposed in a plane perpendicular to or inclined at a predetermined angle with respect to the optical axis L2 so that a part of the filter disc 20 is intersected by the optical axis L2. The filter disc 20 may be placed anywhere in an optical path of the imaging optical system (between the perforated mirror 2 and the photo-receiving element 14) which does not overlap with an optical path of the irradiation optical system.

The filter disc 20 is rotated about a shaft 21a in a direction indicated by an arrow D by a rotator 21 such as a motor. The rotator 21 in the present embodiment is a pulse motor, but it may be a well known motor, encoder, or the like. A sensor 23 for positional detection (rotation angle detection) of the filter disc 20 detects a reference position of the filter disc 20 when the sensor 23 is shielded by a shielding plate 22 provided in a predetermined position of the filter disc 20. The rotation angle of the filter disc 20 is adjusted (controlled) based on the reference position.

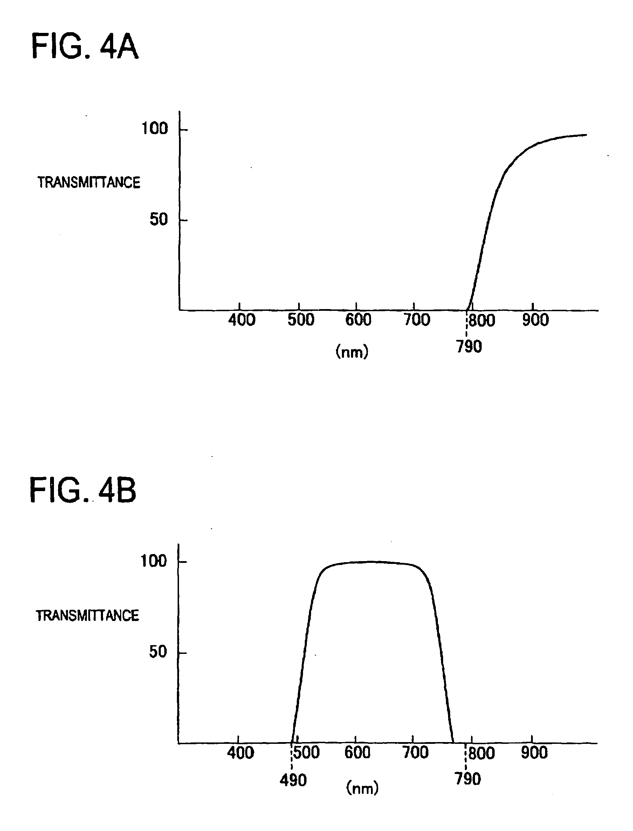

On a rotary disc 20a of the filter disc 20, a first filter 24 and a second filter 25 are attached in place. The first filter 24 has a spectral transmission property as shown in Fig. 4A for infrared-fluorescence fundus imaging such as ICG (indocyanin green fundus angiography). The second filter 25 has a spectral transmission property as shown in Fig. 4B for visible-fluorescence fundus imaging such as FAG (fluorescent fundus angiography). As shown in Fig. 3, each of the first and second filters 24 and 25 is of a substantial sector shape having two arcuate outer lines and two straight outer lines. These filters 24 and 25 are arranged nearly symmetrically with respect to an orthogonal line passing the center of the rotary disc 20a (the shaft 21a) so that the first and second filters 24 and 25 are selectively intersected by (disposed in) the optical path Lz of the imaging optical system that does not overlap with the optical path of the irradiation optical system, that is, the optical axis L2. Those rotary disc 20a, rotator 21, and shaft 21a constitute a filter changer which continuously selectively disposes the first filter 24 and the second filter 25 in the optical path Lz (the optical axis L2) of the imaging optical system.

The first filter 24 intercepts the first and second beams reflected by the fundus Ef and also second fluorescence generated in the fundus Ef when the second beam is irradiated as excitation light. The first filter 24 transmits first fluorescence generated in the fundus Ef when the first beam is irradiated as excitation light. On the other hand, the second filter 25 intercepts the first and second beams reflected by the fundus Ef and the first fluorescence from the fundus Ef and transmits the second fluorescence from the fundus Ef.

The rotary disc 20a of the filter disc 20 is provided with an opening 26 (a single opening is provided in this embodiment, but more than one openings may be provided) between the first and second filters 24 and 25 as shown in Fig. 3. During alignment (positioning) between the apparatus and the eye E, during normal imaging of the fundus Ef, and other cases, the opening 26 is fixedly disposed in the optical path Lz (the optical axis L2) of the imaging optical system to allow all light beams from the fundus Ef to pass therethrough to reach the photo-receiving element 14. The first filter 24, the second filter 25, and the openings 26 are arranged in a predetermined positional relationship relative to the reference position of the filter disc 20.

It is to be noted that the size (the diameter) of the opening 26 is determined to be substantially equal to the size (the diameter) of the optical path Lz of the imaging optical system. In this embodiment, the opening 26 is formed in circular shape (in hole shape), but it may be provided as a cutout partially formed in the rotary disc 20a.

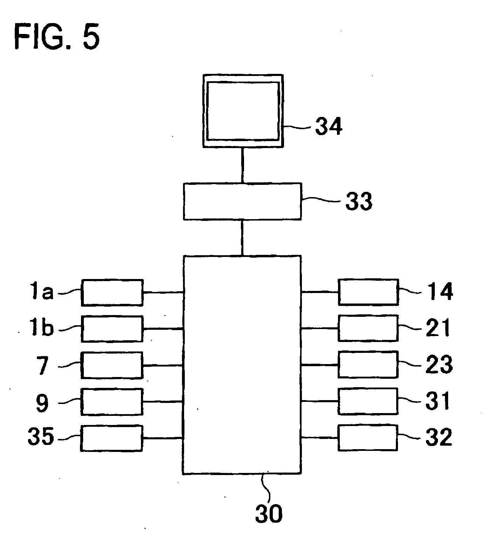

Fig. 5 is a schematic block diagram of a control system of the apparatus.
Connected to an arithmetic control part (control means) 30 which controls all systems of the apparatus are the first laser source 1a, the second laser source 1b, the polygon mirror 7 (its driving part), the galvano mirror 9 (its driving part), the photo-receiving element 14, the rotator 21, the sensor 23, a moving part (a drive part) 31 for moving the mirrors 4 and 5, an operation part 32, an image processing part (image obtaining means) 33 which obtains (generates) an image of the fundus Ef based on an output signal (a received light signal) from the photo-receiving element 14, a memory (a storage part) 35, and others. The fundus image obtained in the image processing part 33 is displayed on a monitor (a display part) 34. The operation part 32 is provided thereon with various switches for operations of the apparatus such as an input part for inputting refractive power of the eye E for diopter movement, an imaging start switch, and a change switch with which the first filter 24, the second filter 25, and the openings 26 are selectively disposed in the optical path Lz.

Operations of the apparatus having the above structure will be described below. Herein, a method of nearly simultaneously performing the ICG and the FAG is explained.

Upon power-on of the apparatus, the control part 30 drives the rotator 21 by default to rotate the filter disc 20 to dispose the opening 26 in the optical path Lz. Further, the first laser source 1a is caused to emit the infrared, first beam as illumination light for observation. When the refractive power data of the eye E previously measured by an eye refractive power measurement apparatus or the like is input by the operation part 32, the control part 30 stores the input refractive power data in the memory 35 while driving the moving part 31 to move the mirrors 4 and 5, thereby performing the diopter movement.

The first beam from the first laser source 1a is scanned in two dimensions on the fundus Ef by the polygon mirror 7 and the galvano mirror 9. The first beam reflected by the fundus Ef is reflected by the perforated mirror 2, passes through the opening 26, comes into focus on the pinhole 12a, and is received by the photo-receiving element 14.

The image processing part 33 generates the image of the fundus Ef based on the received light signal of the photo-receiving element 14 relating the first beam reflected from an area of the fundus Ef scanned by the polygon mirror 7 and the galvano mirror 9. The fundus image is then displayed on the monitor 34. Specifically, images corresponding to one horizontal row are obtained by rotation of one reflection surface of the polygon mirror 7. After each time the images corresponding to the one horizontal row are obtained, the galvano mirror 9 is swung (tilted) downwards stepwise and hence images corresponding to one frame are obtained. When the images corresponding to one frame are obtained, the galvano mirror 9 is returned to an initial upwardly tilted state corresponding to the start of scan, and then the same operation as above is repeated to obtain images corresponding to another frame. In the present embodiment, the infrared, first beam is used as the illumination light for observation, but instead thereof, the visible, second beam may be used.

Upon pressure of the imaging start switch, the control part 30 causes the first laser source 1a to emit the infrared, first beam and the second laser source 1b to emit the visible, second beam. Furthermore, the control part 30 synchronously drives the polygon mirror 7, the galvano mirror 9, and the filter disc 20 respectively to obtain two kinds of desired fluorescent fundus images. The control part 30 further increases the sensitivity (the gain) of the photo-receiving element 14 so that weak fluorescence excited in the fundus Ef can be obtained (received) at a level enough to generate fundus images.

The ICG and FAG are performed at a previously set frame rate and resolution. The frame rate and resolution may be fixed or be set changeably with a setting switch not shown on the operation part 32. The control part 30 rotates the polygon mirror 7 at a rotation speed appropriate to provide the set frame rate and resolution. According to this rotation speed of the polygon mirror 7, the galvano mirror 9 is swung to provide the set frame rate and resolution. By the one-way rotation of the polygon mirror 7 and the one-way swinging (the downward swinging in the present embodiment) of the galvano mirror 9, as mentioned above, the fluorescent fundus images corresponding to one frame to be displayed on the monitor 34 are obtained. The control part 30 rotates the filter disc 20 so that either one of the first filter 24 and the second filter 25 is disposed in the optical path Lz during one-way singing of the galvano mirror 9 to obtain the fluorescent fundus image, and an intermediate portion between the first filter 24 and the second filter 25 is disposed in the optical path Lz during a period from the termination of one scan until the galvano mirror 9 is returned to the initial tilted state corresponding to the scan start, i.e., during the upward swinging of the galvano mirror 9.

The rotation angle of the filter disc 20 can be detected based on a detection signal of the sensor 23 and a pulse signal of the rotator 21, i.e., a pulse motor. The control part 30 controls the rotation of the filter disc 20 so as to maintain the above mentioned relationship with respect to driving of the polygon mirror 7 and the galvano mirror 9. In this way, the intermediate portion between the first filter 24 and the second filter 25 is allowed to pass through the optical path Lz in response to motion (return) of the galvano mirror 9 during which no fluorescent fundus image is obtained. Thus, the fluorescent fundus image can more efficiently be obtained.

The control part 30 can detect which one of the first filter 24, the second filter 25, and the opening 26 is disposed in the optical path Lz (the optical axis L2) based on the rotation angle of the filter disc 20.

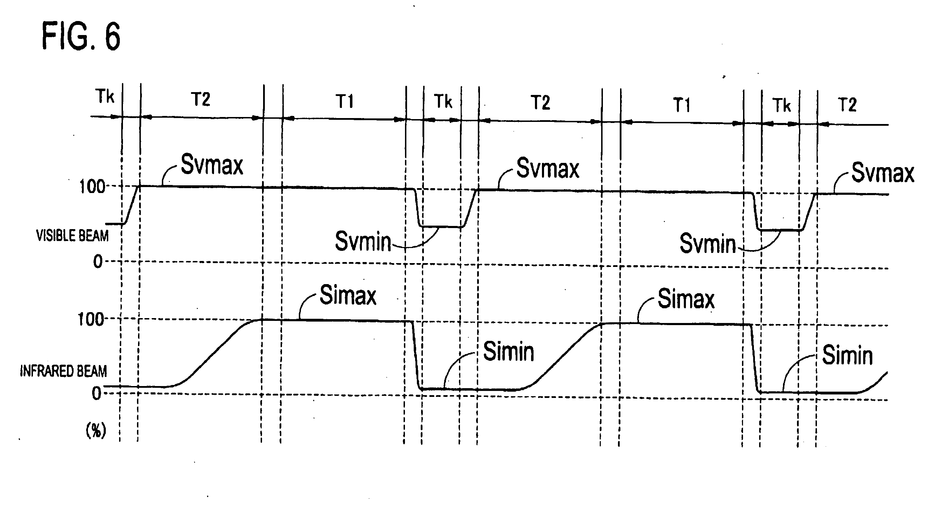

The control part 30 controls the laser emitting part 1 in accordance with the rotation angle of the filter disc 20 (see Fig. 6). Specifically, the control part 30 causes the laser emitting part 1 to emit the first and second beams at respective predetermined power levels. The control part 30 also controls the laser emitting part 1 to reduce each light amount of the first and second beams to be received by the photo-receiving element 14 while the opening 26 is disposed in the optical path Lz. In this case, the control part 30 reduces the power of each of the first and second beams during a period (a time Tk in Fig. 6) from the time when the opening 26 (its outer edge 26a) starts to pass across the optical path Lz to the time when the opening 26 (its outer edge 26b) finishes passing across the optical path Lz after the center of the opening 26 aligns with the optical axis L2. In other words, when the excitation light for fluorescence fundus imaging is to be received by the photo-receiving element 14 after passing through the opening 26, the received light amount of the excitation light is decreased temporarily.

In this embodiment, the filter disc 20 is rotated (in the direction indicated by the arrow D in Fig. 3) to continuously dispose the opening 26, the second filter 25, and the first filter 24 one by one in this order in the optical path Lz.

After a lapse of the time Tk, the control part 30 increases the power of the second beam to a predetermined power level Svₘₐₓ (maximum power for visible-fluorescence imaging) set for the visible-fluorescence imaging before the start of obtaining visible fluorescent fundus images. The control part 30 obtains at least one visible-fluorescent fundus image based on a received light signal from the photo-receiving element 14 during a period (a time T2 in Fig. 6) during which the power of the second beam reaches Svₘₐₓ and the optical path Lz is covered by the second filter 25.

The control part 30 increases the power of the first beam (for example, after a lapse of half of the time T2, in this embodiment) to adjust the power of the first beam to a predetermined power level Siₘₐₓ (maximum power for infrared-fluorescence imaging) set for the infrared-fluorescence imaging before the start of obtaining infrared-fluorescent fundus images. The control part 30 obtains at least one infrared-fluorescent fundus image based on a received light signal from the photo-receiving element 14 during a period (a time T1 in Fig. 6) during which the power of the first beam reaches Siₘₐₓ and the optical path Lz is covered by the first filter 24.

When the visible-fluorescent fundus image and the infrared-fluorescent fundus image are obtained in the above manner, before the opening 26 is disposed in the optical path Lz, the control part 30 decreases the powers of the first and second beams to predetermined power levels Siₘᵢₙ (minimum power for infrared-fluorescence imaging) and Svₘᵢₙ (minimum power for visible-fluorescence imaging) respectively.

When the first and second beams are simultaneously emitted and the opening 26 is disposed in the optical path Lz, the control part 30 raises a decreasing rate of the power of the first beam than a decreasing rate of the power of the second beam to reduce the light amounts of the first and second beams to be received by the photo-receiving element 14. In this way, the rate of decreasing power of the first laser source 1a (e.g. 90% or more) is set higher than the rate of decreasing power of the second laser source 1b (e.g. about 50%) is as follows. This is because the power level Siₘₐₓ of the infrared, first beam needs to be set higher than the power level Svₘₐₓ of the visible, second beam in order to raise the accuracy in obtaining weak infrared first fluorescence generated in the fundus Ef and hence the power of the first beam has to be reduced largely to avoid saturation of the photo-receiving element 14.

In the case where the power of the first beam is to be reduced largely as above, it will take long for the power of the first beam to return to the power level Siₘₐₓ needed for infrared-fluorescence imaging. In the present embodiment, the filter disc 20 is configured such that the opening 26, the second filter 25, and the first filter 24 are repeatedly disposed one by one in the optical path Lz. Accordingly, it is possible to increase and return the power of the first beam to Siₘₐₓ while the visible-fluorescence imaging is performed with the second beam and the second filter 25, so that the visible-fluorescence imaging and the infrared-fluorescence imaging can be performed smoothly.

When a fluorescent agent for the ICG and a fluorescent agent for the FAG are circulated in the fundus Ef, the fluorescent fundus image by the infrared, first beam and the fluorescent fundus image by the visible, second beam are obtained. Specifically, when the first filter 24 is disposed in the optical path Lz by rotation of the filter disc 20, only the first fluorescence from the fundus Ef, resulting from the first beam, reaches the photo-receiving element 14. When the second filter 25 is disposed in the optical path Lz, only the second fluorescence from the fundus Ef, resulting from the second beam, reaches the photo-receiving element 14. Consequently, each fluorescent fundus image can be obtained successively in timesharing manner.

The image processing part 33 obtains a fundus image based on the received light signal from the photo-receiving element 14 via the control part 30 when the first filter 24 is disposed in the optical path Lz, and then displays the image as a fundus image by the ICG on the monitor 34. Further, the image processing part 33 obtains a fundus image based on the received light signal from the photo-receiving element 14 via the control part 30 when the second filter 25 is disposed in the optical path Lz, and then displays the image as a fundus image by the FAG on the monitor 34. In the present embodiment, the ICG fundus image and the FAG fundus image are displayed side by side on the monitor 34. Preferably, information for distinguishing them is also displayed. As an alternative, only one of the fundus images may be selectively displayed on the monitor 34 with a switch not shown on the operation part 32.

During rotation of the filter disc 20, the first filter 24 and the second filter 25 are continuously selectively disposed in the optical path Lz. Consequently, the ICG fundus image and the FAG fundus image can be obtained at substantially the same time (alternately every one frame). The variations thereof with time can be displayed as moving images on the monitor 34.

The apparatus, which is arranged to use the visible, second beam as illumination light (excitation light) for visible-fluorescence fundus imaging and scan the second beam in two dimensions as described above, enables the visible-fluorescence fundus imaging without causing glare to an examinee. Since the infrared, first beam and the visible, second beam are emitted simultaneously, respective fluorescent fundus images can be obtained at almost the same time, thus reducing a time needed for inspection (medical examination). Further, a single photo-receiving element receives the first fluorescence resulting from the infrared, first beam and the second fluorescence resulting from the visible, second beam. This makes it possible to reduce the number of optical components, substrate parts, and others. Accordingly, a downsized apparatus can be realized.

The beam emitting part 1 is controlled as above to avoid saturation of the photo-receiving element 14 by the first and second beams directly passing through the opening 26. Accordingly, an appropriate fluorescent fundus image can be obtained efficiently.

In the case where respective powers of the first and second beams are to be reduced as mentioned above while the opening 26 is disposed in the optical path Lz, the powers of the first and second beams have only to be controlled (reduced) to provide such a received light amount as not to saturate the photo-receiving element 14. While the first filter 24 or the second filter 25 is disposed in the optical path Lz, the powers of the first and second beams have only to be controlled (increased or reduced) to provide a received light amount needed for obtaining each fluorescent fundus image.

Furthermore, the filter disc 20 is preferably rotated at such a speed as to allow the above control of the powers of the first and second beams in response to successive selective disposing of the first filter 24, the second filter 25, and the opening 26 in the optical path Lz.

In the above embodiment, the first and second laser sources 1a and 1b are controlled by the control part 30 to reduce the powers of the first and second beams. However, it is only necessary to reduce the respective amounts of the first and second beams to be received by the photo-receiving element 14. For instance, a shutter mechanism may be provided including a light shielding plate for shielding the first and second beams and a moving part (a driving part) for moving the light shielding plate in or out of the optical path Lz. The moving part is controlled by the control part 30 so as to move into the optical path Lz in association with disposing of the opening 26 in the optical path Lz to reduce the respective received light amounts of the first and second beams.

Although the apparatus in the above embodiment is arranged to perform one manner of infrared-fluorescence fundus imaging and one manner of visible-fluorescence fundus imaging, it may be arranged to perform a plurality of manners of fluorescence fundus imaging. In such cases, the type and the number of filters provided in the filter disc 20 may be determined according to the types and the number, or the like, of the laser sources provided in the laser emitting part 1.

While the presently preferred embodiment of the present invention has been shown and described, it is to be understood that this disclosure is for the purpose of illustration and that various changes and modifications may be made without departing from the scope of the invention as set forth in the appended claims.

## Claims

1. A fundus imaging apparatus comprising:
a beam emitter (1) adapted to simultaneously.emit a first laser beam and a second laser beam having a different wavelength from the first beam;
an irradiation optical system having a beam scanner (7, 9) for scanning the emitted first and second beams in two dimensions on a fundus (Ef), the irradiation optical system being adapted to irradiate the emitted first and second beams onto the fundus;
a filter disc (20) having a first filter (24) which intercepts the first and second beams reflected by the fundus and second fluorescence from the fundus by irradiation of the second beam and transmits first fluorescence from the fundus by irradiation of the first beam, a second filter (25) which intercepts the first and second beams reflected by the fundus and the first fluorescence from the fundus and transmits the second fluorescence from the fundus, and an opening (26); an imaging optical system having the filter disc and a photo-receiving element (14);
a rotator (21) for rotating the filter disc to dispose one of the first filter, the second filter, and the opening in an optical path (Lz) of the imaging optical system;
a sensor (23) for detecting a rotation angle of the filter disc;
control means (30) for controlling the beam emitter to simultaneously emit the first and second beams at respective predetermined powers; and
image processing means (33) for obtaining a first fluorescent fundus image based on a received light signal of the first fluorescence from the photo-receiving element and obtaining a second fluorescent fundus image based on a received light signal of the second fluorescence from the photo-receiving element, **characterized by** the control means being adapted to control at least one of the beam emitter, the irradiation optical system, and the imaging optical system based on a detection signal of the sensor to reduce respective light amounts of the first and second beams to be received by the photo-receiving element while the opening is disposed in the optical path of the imaging optical system.

2. The fundus imaging apparatus according to claim 1, wherein
the first beam is an infrared beam,
the second beam is a visible beam, and
the control means controls at least one of the beam emitter, the irradiation optical system, and the imaging optical system to decrease the light amounts of the first and second beams to be received by the photo-receiving element, the decreasing rate of the light amount of the first beam is larger than the decreasing rate of the light amount of the second beam while the opening is disposed in the optical path of the imaging optical system.

3. The fundus imaging apparatus according to claim 2, wherein
a maximum power of the first beam for obtaining the first fluorescent fundus image is set higher than a maximum power of the second beam for obtaining the second fluorescent fundus image.

4. The fundus imaging apparatus according to claim 1, wherein
the first beam is an infrared beam, the second beam is a visible beam, and
the filter disc is adapted to rotate to repeatedly selectively dispose the opening, the second filter, and the first filter in this order in the optical path of the imaging optical system.

5. The fundus imaging apparatus according to one of claims 1 to 4, wherein
the control means controls the rotator to continuously selectively dispose the first filter and the second filter in the optical path of the imaging optical system in sync with scanning of the beam scanner.

6. The fundus imaging apparatus according to one of claims 1 to 5, wherein
the photo-receiving element is a single photo-receiving element adapted to receive the first and second beams and the first and second fluorescences.

## Patentansprüche

1. Vorrichtung zum Abbilden eines Augenhintergrunds, mit:
einem Strahlaussender (1), der daran angepaßt ist, gleichzeitig einen ersten Laserstrahl und einen zweiten Laserstrahl auszusenden, der eine andere Wellenlänge als der erste Strahl hat;
einem optischen Bestrahlungssystem mit einem Strahlscanner (7, 9) zum Scannen des ausgesendeten ersten und zweiten Strahls in zwei Dimensionen an einem Augenhintergrund (Ef), wobei das optische Strahlungssystem daran angepaßt ist, den ausgesendeten ersten und zweiten Strahl auf den Augenhintergrund zu strahlen;
einer Filterscheibe (20) mit einem ersten Filter (24), der den ersten und zweiten Strahl, die durch den Augenhintergrund reflektiert werden, und eine zweite Fluoreszenz von dem Augenhintergrund durch Bestrahlung des zweiten Strahls abfängt und eine erste Fluoreszenz von dem Augenhintergrund durch Bestrahlung des ersten Strahls durchläßt, einem zweiten Filter (25), der den ersten und zweiten Strahl, die durch den Augenhintergrund reflektiert werden, und die erste Fluoreszenz von dem Augenhintergrund abfängt und die zweite Fluoreszenz von dem Augenhintergrund durchläßt, und einer Öffnung (26);
einem optischen Abbildungssystem mit der Filterscheibe und einem Lichtaufnahmeelement (14);
einem Rotor (21) zum Drehen der Filterscheibe, um entweder den ersten Filter, den zweiten Filter oder die Öffnung in einen optischen Pfad (Lz) des optischen Abbildungssystems anzuordnen;
einem Sensor (23) zum Erfassen eines Drehwinkels der Filterscheibe;
einer Steuereinrichtung (30) zum Steuern des Strahlaussenders, um gleichzeitig den ersten und den zweiten Strahl mit jeweils vorbestimmten Leistungen auszusenden; und
einer Bildverarbeitungseinrichtung (33) zum Erhalten eines ersten Fluoreszenzaugenhintergrundbildes auf der Grundlage eines aufgenommenen Lichtsignals der ersten Fluoreszenz von dem Lichtaufnahmeelement und zum Erhalten eines zweiten Fluoreszenzaugenhintergrundbildes auf der Grundlage eines aufgenommenen Lichtsignals der zweiten Fluoreszenz von dem Lichtaufnahmeelement, **gekennzeichnet durch**
die Steuereinrichtung, die daran angepaßt ist, zumindest den Strahlaussender, das optische Bestrahlungssystem oder das optische Abbildungssystem auf der Grundlage eines Erfassungssignals von dem Sensor zu steuern, um entsprechende Lichtmengen des ersten und des zweiten Strahls zu reduzieren, die **durch** das Lichtaufnahmeelement aufzunehmen sind, während die Öffnung in dem optischen Pfad des optischen Abbildungssystems angeordnet ist.

2. Vorrichtung zum Abbilden eines Augenhintergrunds gemäß Anspruch 1, wobei
der erste Strahl ein Infrarotstrahl ist,
der zweite Strahl ein sichtbarer Strahl ist, und
die Steuereinrichtung zumindest den Strahlaussender, das optische Bestrahlungssystem oder das optische Abbildungssystem zum Verringern der Lichtmengen des ersten und zweiten Strahls steuert, die durch das Lichtaufnahmeelement aufzunehmen sind, wobei die Verringerungsrate der Lichtmenge des ersten Strahls größer ist als die Verringerungsrate der Lichtmenge des zweiten Strahls, während die Öffnung in dem optischen Pfad des optischen Abbildungssystems angeordnet ist.

3. Vorrichtung zum Abbilden eines Augenhintergrunds gemäß Anspruch 2, wobei
die maximale Leistung des ersten Strahls zum Erhalten des ersten Fluoreszenzaugenhintergrundbildes höher festgelegt ist als eine maximale Leistung des zweiten Strahls zum Erhalten des zweiten Fluoreszenzaugenhintergrundbildes.

4. Vorrichtung zum Abbilden eines Augenhintergrunds gemäß Anspruch 1, wobei
der erste Strahl ein Infrarotstrahl ist, der zweite Strahl ein sichtbarer Strahl ist, und
die Filterscheibe daran angepaßt ist, sich so zu drehen, dass wahlweise die Öffnung, der zweite Filter und der erste Filter in dieser Reihenfolge in den optischen Pfad des optischen Abbildungssystems wiederholt angeordnet werden.

5. Vorrichtung zum Abbilden eines Augenhintergrunds gemäß einem der Ansprüche 1 bis 4, wobei
die Steuereinrichtung den Rotor zum kontinuierlichen und wahlweisen Anordnen des ersten Filters und des zweiten Filters in den optischen Pfad des optischen Abbildungssystems synchron mit dem Scannen des Strahlscanners steuert.

6. Vorrichtung zum Abbilden eines Augenhintergrunds gemäß einem der Ansprüche 1 bis 5, wobei
das Lichtaufnahmeelement ein einziges Lichtaufnahmeelement ist, das daran angepaßt ist, den ersten und den zweiten Strahl sowie die erste und die zweite Fluoreszenz aufzunehmen.

## Revendications

1. Appareil d'imagerie du fond de l'oeil comprenant :
un émetteur de faisceau (1) adapté pour émettre simultanément un premier faisceau laser et un deuxième faisceau laser ayant une longueur d'onde différente du premier faisceau ;
un système optique de rayonnement ayant un scanner de faisceau (7, 9) pour balayer les premier et deuxième faisceaux émis en deux dimensions sur un fond de l'oeil (Ef), le système optique de rayonnement étant adapté pour rayonner les premier et deuxième faisceaux émis sur le fond de l'oeil ;
un disque filtrant (20) ayant un premier filtre (24) qui intercepte les premier et deuxième faisceaux réfléchis par le fond de l'oeil et la deuxième fluorescence provenant du fond de l'oeil par rayonnement du deuxième faisceau et transmet la première fluorescence provenant du fond de l'oeil par rayonnement du premier faisceau, un deuxième filtre (25) qui intercepte les premier et deuxième faisceaux réfléchis par le fond de l'oeil et la première fluorescence provenant du fond de l'oeil et transmet la deuxième fluorescence provenant du fond de l'oeil, et une ouverture (26) ;
un système optique d'imagerie ayant le disque filtrant et un élément photorécepteur (14) ;
un rotateur (21) pour faire tourner le disque filtrant afin de disposer l'un parmi le premier filtre, le deuxième filtre et l'ouverture dans une trajectoire optique (Lz) du système optique d'imagerie ;
un capteur (23) pour détecter un angle de rotation du disque filtrant ;
des moyens de commande (30) pour commander l'émetteur de faisceau afin qu'il émette simultanément les premier et deuxième faisceaux à des puissances prédéterminées respectives ; et
des moyens de traitement d'image (33) pour obtenir une première image de fond de l'oeil fluorescente basée sur un signal de lumière reçu de la première fluorescence provenant de l'élément photorécepteur et pour obtenir une deuxième image de fond de l'oeil fluorescente basée sur un signal de lumière reçu de la deuxième fluorescence provenant de l'élément photorécepteur, **caractérisé par** :
les moyens de commande qui sont adaptés pour commander au moins l'un parmi l'émetteur de faisceau, le système optique de rayonnement et le système optique d'image en fonction d'un signal de détection du capteur afin de réduire la réception des quantités de lumière respectives des premier et deuxième faisceaux par l'élément photorécepteur alors que l'ouverture est disposée dans la trajectoire optique du système optique d'imagerie.

2. Appareil d'imagerie de fond de l'oeil selon la revendication 1, dans lequel :
le premier faisceau est un faisceau infrarouge,
le deuxième faisceau est un faisceau visible, et
les moyens de commande commandent au moins l'un parmi l'émetteur de faisceau, le système optique de rayonnement et le système optique d'imagerie pour réduire la réception des quantités de lumière des premier et deuxième faisceaux par l'élément photorécepteur, la vitesse de réduction de la quantité de lumière du premier faisceau est supérieure à la vitesse de réduction de la quantité de lumière du deuxième faisceau alors que l'ouverture est disposée dans la trajectoire optique du système optique d'imagerie.

3. Appareil d'imagerie de fond de l'oeil selon la revendication 2, dans lequel :
une puissance maximum du premier faisceau pour obtenir la première image de fond de l'oeil fluorescente est supérieure à une puissance maximum du deuxième faisceau pour obtenir la deuxième image de fond de l'oeil fluorescente.

4. Appareil d'imagerie de fond de l'oeil selon la revendication 1, dans lequel :
le premier faisceau est un faisceau infrarouge, le deuxième faisceau est un faisceau visible, et
le disque filtrant est adapté pour tourner afin de disposer de manière sélectivement répétée l'ouverture, le deuxième filtre et le premier filtre dans cet ordre dans la trajectoire optique du système optique d'imagerie.

5. Appareil d'imagerie de fond de l'oeil selon l'une quelconque des revendications 1 à 4, dans lequel les moyens de commande commandent le rotateur pour disposer de manière sélectivement continue le premier filtre et le deuxième filtre dans la trajectoire optique du système optique d'imagerie en synchronisation avec le balayage du scanner de faisceau.

6. Appareil d'imagerie de fond de l'oeil selon l'une quelconque des revendications 1 à 5, dans lequel l'élément photorécepteur est un élément photorécepteur unique adapté pour recevoir les premier et deuxième faisceaux et les première et deuxième fluorescences.
